# EUROPEAN PATENT APPLICATION

(11) **EP 4 427 734 A1**
(43) Date of publication of application: **11.09.2024**
(21) Application number: 22903018.4
(22) Date of filing: 18.10.2022
(51) Int. Cl.: A61K 8/9794, A61K 8/49, A61Q 19/08, A61Q 19/00

(54) **COMPOSITION FOR PROMOTING ANTIOXIDASE EXPRESSION AND USE THEREOF**

(30) Priority: 10.12.2021 CN 202111509991
(71) Applicant: Inertia Shanghai Biotechnology Co., Ltd., Shanghai 200041 (CN); Dermahealth Shanghai Biotechnology Co., Ltd., Shanghai 200072 (CN)
(72) Inventor: YE, Rui, Shanghai 200041 (CN); HU, Fan, Shanghai 200041 (CN); HU, Xiaoyun, Shanghai 200041 (CN)
(74) Representative: Balder IP Law, S.L.
(86) International application number: PCT/CN2022/125930
(87) International publication number: WO 2023/103594

(57) **Abstract**

The embodiments of the present disclosure relate to the technical field of cosmetics, and disclose a composition capable of enhancing an expression efficacy of an antioxidant enzyme and use thereof. The composition includes a Curcuma Longa (Turmeric) root extract and dimethylmethoxy chromanol, wherein a mass fraction ratio of the Curcuma Longa (Turmeric) root extract to the dimethylmethoxy chromanol is (0.5-3): 1. A synergistic effect can be achieved in terms of promoting the expression efficacy of an antioxidant enzyme by mixing the Curcuma Longa (Turmeric) root extract and the dimethylmethoxy chromanol at a specific mass fraction ratio. Therefore, the use of the composition according to the present disclosure with the effect of promoting the expression of antioxidant enzymes in a cosmetics product and/or a skin care product exerts significant skin antioxidant and anti-aging effects at a lower application amount.

## Description

### CROSS-REFERENCE TO RELATED APPLICATION

This application claims priority to Chinese Patent Application No. 202111509991.2, filed on December 10, 2021, which is incorporated herein by reference in its entirety.

### TECHNICAL FIELD

Embodiments of the present disclosure relate to the technical field of cosmetics, and in particular, to a composition capable of enhancing an expression efficacy of an antioxidant enzyme and use thereof.

### BACKGROUND

In the process of body metabolism, where one electron of oxygen is abstracted, an oxygen atom without paired electron becomes a free radical. Free radicals are unstable, have strong oxidation, and have a very strong ability to absorb electrons. Free radicals need to take electrons again in the cell molecules of body tissues to pair themselves. When cell molecules expel new electrons, the cell molecules also become free radicals, and need to seize the electrons in the cell membranes or nucleus molecules, thus generating new free radicals. Free radicals can cause protein denaturation and cross-linking, such that many enzymes and hormones in the body lose biological activity, the body's immune ability, nerve reflex ability, exercise ability and other system activity are reduced. At the same time, the structure of nucleic acid is destroyed and metabolic disorders are caused to the whole body, and finally lesions are caused to the body.

Under the action of enzyme, a considerable part of oxygen inhaled from the air into human body is converted into superoxide anions, which are used to eliminate external invaders such as virus and hence protect the body. However, the excess superoxide anions become free radicals which promote the aging of human cells. The human body itself can produce endogenous antioxidant substances to catalyze the decomposition of superoxide anions, i.e., free radicals, such that the human body comes to a safe equilibrium state. However, with the increase of age, the level of endogenous antioxidant produced by the human body gradually decreases. At this time, excess superoxide anions, i.e., free radicals, may attack the unsaturated phospholipids on the cell membranes, such that the cells prematurely age, pigment deposition is caused and senile plaques are formed, leading to human aging.

### SUMMARY

The embodiments of the present disclosure are intended to provide a composition capable of enhancing expression efficacy of antioxidant enzyme and use thereof.

According to a first aspect of the embodiments of the present disclosure, a composition capable of enhancing an expression efficacy of an antioxidant enzyme is provided. The composition includes: a Curcuma Longa (Turmeric) root extract and dimethylmethoxy chromanol, wherein a mass fraction ratio of the Curcuma Longa (Turmeric) root extract to the dimethylmethoxy chromanol is (0.5-3): 1.

Preferably, in the composition according to the embodiments of the present disclosure, the mass fraction ratio of the Curcuma Longa (Turmeric) root extract to the dimethylmethoxy chromanol is (1-2): 1.

Preferably, in the composition according to the embodiments of the present disclosure, the mass fraction ratio of the Curcuma Longa (Turmeric) root extract to the dimethylmethoxy chromanol is 1:1.

According to a second aspect of the embodiments of the present disclosure, use of the composition capable of enhancing the expression efficacy of the antioxidant enzyme according to the first aspect in a cosmetics product and/or a skin care product is provided.

In the use according to the embodiments of the present disclosure, the cosmetics product and/or the skin care product is applied to an outer surface of skin.

Preferably, in the use according to the embodiments of the present disclosure, the cosmetics product and/or the skin care product is selected from one or more of eye cream, face cream, essence, lotion, and mask essence.

Preferably, in the use according to the embodiments of the present disclosure, the composition capable of enhancing the expression efficacy of the antioxidant enzyme is dispersed as an additive in a base material selected from one or more of an oily material, a powdery material, and a colloidal material.

Preferably, in the use according to the embodiments of the present disclosure, the cosmetics product and/or the skin care product further includes: one or more of a surfactant, a perfume and fragrance, a pigment, a humectant, a preservative, an antioxidant, a UV absorber, a chelating agent, an astringent, a penetration enhancer, and a nutritional additive.

With respect to the related art, in the embodiments of the present disclosure, a synergistic effect can be achieved in terms of promoting the expression efficacy of an antioxidant enzyme by mixing the Curcuma Longa (Turmeric) root extract and the dimethylmethoxy chromanol at a specific mass fraction ratio. Therefore, the use of the composition according to the embodiments of the present disclosure with the effect of promoting the expression of antioxidant enzymes in a cosmetics product and/or a skin care product exerts significant skin antioxidant and anti-aging effects at a lower application amount.

### BRIEF DESCRIPTION OF THE DRAWINGS

FIG 1 is a graph of comparison of effects of a blank control group, a model group, a separate administration group 1, a separate administration group 2, and an experimental group 1 on UVB-induced SOD activity of Hacat cells;
FIG 2 is a graph of comparison of effects of a blank control group, a model group, a separate administration group 1, a separate administration group 2, and an experimental group 2 on UVB-induced SOD activity of Hacat cells;
FIG 3 is a graph of comparison of effects of a blank control group, a model group, a separate administration group 1, a separate administration group 2, and an experimental group 3 on UVB-induced SOD activity of Hacat cells;
FIG 4 is a WB graph of NQO1 protein expression of UVB-induced Hacat cells in a blank control group, a model group, a separate administration group 1, a separate administration group 2, and an experimental group 1;
FIG 5 is a graph of comparison of effects of a blank control group, a model group, a separate administration group 1, a separate administration group 2, and an experimental group 1 on NQO1 protein expression of UVB-induced Hacat cells;
FIG 6 is a WB graph of NQO1 protein expression of UVB-induced Hacat cells in a blank control group, a model group, a separate administration group 1, a separate administration group 2, and an experimental group 2;
FIG 7 is a graph of comparison of effects of a blank control group, a model group, a separate administration group 1, a separate administration group 2, and an experimental group 2 on NQO1 protein expression of UVB-induced Hacat cells;
FIG 8 is a WB graph of NQO1 protein expression of UVB-induced Hacat cells in a blank control group, a model group, a separate administration group 1, a separate administration group 2, and an experimental group 3; and
FIG 9 is a graph of comparison of effects of a blank control group, a model group, a separate administration group 1, a separate administration group 2, and an experimental group 3 on NQO1 protein expression of UVB-induced Hacat cells.

### DETAILED DESCRIPTION OF THE EMBODIMENTS

For clearer descriptions of the objects, technical solutions, and advantages of embodiments of the present disclosure, the embodiments of the present disclosure are described in detail hereinafter. However, persons of ordinary skill in the art may understand, in the embodiments of the present disclosure, more technical details are provided for readers to better understand the present disclosure. However, even though these technical details and various variations and modifications based on the embodiments hereinafter, the technical solutions of the present disclosure may also be practiced.

Some embodiments of the present disclosure provide a composition capable of enhancing an expression efficacy of an antioxidant enzyme. The composition includes a Curcuma Longa (Turmeric) root extract and dimethylmethoxy chromanol, where a mass fraction ratio of the Curcuma Longa (Turmeric) root extract to the dimethylmethoxy chromanol (i.e., the ratio of the mass fraction of the Curcuma (Tumeric) root extract to the mass fraction of the dimethylmethoxy chromanol) is (0.5-3): 1.

Some embodiments of the present disclosure provide a composition capable of enhancing an expression efficacy of an antioxidant enzyme. The composition includes a Curcuma Longa (Turmeric) root extract and dimethylmethoxy chromanol, where a mass fraction ratio of the Curcuma Longa (Turmeric) root extract to the dimethylmethoxy chromanol is (1-2): 1.

Some embodiments of the present disclosure provide a composition capable of enhancing an expression efficacy of an antioxidant enzyme. The composition includes a Curcuma Longa (Turmeric) root extract and dimethylmethoxy chromanol, where a mass fraction ratio of the Curcuma Longa (Turmeric) root extract to the dimethylmethoxy chromanol is 1: 1.

The composition capable of enhancing the expression efficacy of the antioxidant enzyme according to the embodiments of the present disclosure is applicable to a cosmetics product and/or a skin care product, where the cosmetics product and/or the skin care product is applied to an outer surface of skin.

In some embodiments of the present disclosure, the cosmetics product and/or the skin care product utilizing the composition with the efficacy of enhancing antioxidant enzyme expression is selected from one or more of eye cream, face cream, essence, lotion, and mask essence.

In some embodiments of the present disclosure, the composition with the efficacy of promoting antioxidant enzyme expression is dispersed as an additive in a base material selected from one or more of an oily material, a powdery material, a colloidal material, an oil and fat material, and a solvent material.

The oily feedstock may be any one or combination of matrix materials selected from: soybean oil, olive oil, almond oil, castor oil, peanut oil, cottonseed oil, tea seed oil, jojoba oil, avocado oil, coconut oil, palm oil, cocoa butter, rice bran oil, evening primrose oil, wheat germ oil, corn germ oil, palm wax, wood wax, mink oil, turtle oil, snake oil, egg yolk oil, tallow, horse fat, lard, deer fat, lecithin, lanolin, beeswax, spermaceti, shellac wax, paraffin, petrolatum, microcrystalline wax, ozokerite, squalane, lanolin wax, lanolin alcohol, lanolin alcohol ester, acetylated lanolin, acetylated lanolin alcohol, lanolin acid, lanolin acid ester, polyoxyethylene lanolin, polyoxyethylene lanolin alcohol ether, polyoxypropylene lanolin alcohol ether, hydrogenated lanolin, alkoxylated hydrogenated lanolin, silicone oils (e.g., dimethyl silicone oil, octamethyl silicone oil, methylphenyl polysiloxane), polysiloxane-polyoxyalkyl block copolymers, methyl hydrogen silicone oil, Cn fatty acids and esters thereof, and Cn fatty alcohols and esters thereof; wherein n≤10, and preferably n is 10 to 24, more preferably from 12 to 20, such as 14, 16, 17, or 18.

The Cn fatty acid is selected from: lauric acid, myristic acid, palmitic acid, stearic acid, oleic acid, linoleic acid, erucic acid, and capric acid. The Cn fatty alcohol is selected from: lauryl alcohol, cetyl alcohol, stearyl alcohol, isostearyl alcohol, oleyl alcohol, and coco alcohol. The Cn fatty acid ester and Cn fatty alcohol ester are selected from: isopropyl myristate, myristyl myristate, myristyl lactate, cetyl lactate, isopropyl palmitate, isooctyl palmitate, butyl stearate, isooctyl stearate, glyceryl monostearate, polyethylene glycol stearate, decyl oleate, glyceryl caprylate, glyceryl caprate, and glyceryl trioleate.

The powdery material may be any one or a combination of matrix materials selected from: mica, magnesium carbonate, titanium dioxide, zinc white powder, calcium hydroxide, calcium carbonate, kaolin, calcium stearate, zinc stearate, magnesium stearate, talc, silica, aluminum hydroxide, calcium pyrophosphate, calcium bicarbonate, and bentonite.

The oil and fat material may be any one or more of a vegetable oil material, an animal oil material, a mineral oil material, a synthetic oil material, and a semi-synthetic oil material. For example, starch, cyclodextrin, xanthan gum, carrageenan, guar gum, gum arabic, tragacanth, agar, shellac, sodium alginate, gelatin, methyl cellulose, ethyl cellulose, sodium carboxymethyl cellulose, hydroxyethyl cellulose, hydroxypropyl cellulose, poly cellulose ether quaternary ammonium salts, polyvinyl alcohol, polyvinyl pyrrolidone, sodium polyacrylate, polyoxyethylene, vinyl pyrrolidone/vinyl acetate copolymer, carbopol resin, magnesium aluminum silicate, and C12-15 alcohol benzoate.

The solvent material can be any one or a combination of matrix materials selected from: water, ethanol, propanol, isopropanol, isobutanol, ethylene glycol, 1,2-propylene glycol, cyclohexane, dichlorodifluoromethane, tetrafluoro ethylene dichloride, ethylene glycol monoethyl ether, diethylene glycol monoethyl ether, acetone, methyl isobutyl ketone, ethyl acetate, butyl acetate, amyl acetate, and dibutyl phthalate.

In some embodiments of the present disclosure, the cosmetic and/or skin care product to which the composition having the effect of promoting expression of an antioxidant enzyme is applied further includes: one or more of surfactants, perfumes and fragrances, pigments, humectants, preservatives, antioxidants, UV absorbers, chelating agents, astringents, penetration enhancers, pH regulators, and nutritional additives.

The surfactant may be selected from: 1) anionic surfactants such as C12-14 fatty alcohol ammonium sulfate, C12-14 fatty alcohol polyoxyethylene ether carboxylate, diethanolamine lauryl sulfate, ammonium C12-14 fatty alcohol polyoxyethylene ether sulfate, triethanolamine lauryl sulfate, alcohol ether phthalic acid monoester sodium salt, acylated peptide, lauric acid monoethanolamide sodium sulfate, n-octanol polyoxypropylene ether succinic acid monoester sulfonate, cetyl alcohol succinic acid monoester sulfonate, sulfosuccinic acid monoester disodium salt, succinic acid monooctadecyl ester disodium salt, alcohol ether sulfosuccinic acid monoester disodium salt, fatty alcohol polyoxyethylene ether (sodium) sulfosuccinic acid monoester ammonium salt, oleamide sulfosuccinic acid monoester disodium salt, N-acyl glutamic acid potassium salt, sodium pyrrolidone carboxylate, sodium polyacrylate, fatty alcohol polyoxyethylene ether phosphate, fatty alcohol polyoxyethylene ether phosphate monoester and salts thereof, fatty alcohol polyoxyethylene ether phosphate monoester ethanolamine salt, alkylphenol polyoxyethylene ether phosphate monoester and salts thereof, nonylphenol ether phosphate monoester ethanolamine salt, alkyl phosphate ester salt, monolauryl phthalate ester salt, zinc undecylenate, anionic amino acid surfactant, sodium N-C12-18 acyl glutamate, sodium N-lauroyl L-alaninate, N-stearoyl glutamic acid monosodium salt, N-cocoyl glutamic acid monosodium salt, N-mixed fatty acyl glutamic acid monosodium salt, and sodium long linear alkyl aryl ether sulfonate TH; 2) non-ionic surfactants such as oleyl alcohol polyoxyethylene ethers (such as the emulsifier VO series), alkylphenol polyoxyethylene ethers (such as the emulsifier OPE series), polyoxyethylene stearates, ethylene glycol monostearate, ethylene glycol distearate, polyethylene glycol monostearate, polyethylene glycol distearate, polyethylene glycol didodecanoate, glyceryl distearate, glyceryl tristearate, sucrose stearate, polyoxyethylene glyceryl ether monostearate, dialkanol N-lauroyl glutamate, fatty alcohol benzoate, sorbitan monofatty acid esters (span, such as span-20, span 60, span 65, span-80, span-83, span-85), polyoxyethylene sorbitan mono-fatty acid esters (tween, such as tween-20, tween-40, tween-60, tween-61, tween-80), diethanolamine, triethanolamine, fatty acid monoethanolamides (such as coconut acid monoethanolamide, lauric acid monoethanolamide, palmitic acid monoethanolamide), coconut acid diethanolamide, lauric acid diethanolamide, methyl glucoside stearate, polyoxyethylene methyl glucoside stearate, ethylene glycol glucoside stearate, propylene glycol glucoside stearate, C8-16 alkyl glucoside, glycerol glucoside stearate, lauryl alcohol ether monoester of succinic acid, lauryl alcohol glyceride polyoxyethylene ether, mannitol anhydride stearate, polyoxyethylene mink oil, methyl mink oleate, and isopropyl mink oleate; 3) cationic surfactants, such as C12-18 alkyl trialkyl ammonium chlorides (e.g. cetyl dimethyl alkyl ammonium chloride, octadecyl trimethyl ammonium chloride, cetyl trimethyl ammonium chloride), oleamidopropyl dihydroxypropyl dimethyl ammonium chloride, dihydroxypropyl dimethyl dodecyl ammonium chloride, dimethyl diallyl ammonium chloride/acrylamide copolymer, oleamidopropyl-2,3-dihydroxypropyl dimethyl ammonium chloride, oleamidopropyl dimethylethyl ammonium ethylsulfate, di-C12-18 alkyl dimethyl ammonium chloride, polyquaternium -11 conditioner, M-505 polyquaternium-7 conditioning agent, minkoleamidopropylamine-chitosan, minkoleamidopropylamine-hydrolyzed protein, cationic protein peptide, guar gum-hydroxypropyl trimethyl ammonium chloride salt, polycellulose ether quaternary ammonium salts, triethanolamine monooleate, and DNP series; 4) zwitterionic surfactants such as C12-18 alkyl dihydroxyethyl betaines, N-C12-18 alkyl-N-(2-hydroxyethyl)-N-(2-formamidoethyl) ammonium acetate, C12-18 alkoxy hydroxypropyl betaine, cocamidopropylamine oxide, glycol monostearate, octadecyldimethylamine oxide, coco diethanolamine amine oxide, N-alkyl-beta-aminopropionyl diethanolamine, lauroylpropylamine oxide, N-C12-18 acylglutamic acid, hydroxyethyl capric acid imidazoline betaine, hydroxyethyl myristic acid imidazoline betaine, and hydroxyethyl palmitoyl imidazolinium betaines.

The flavors and fragrances may be selected from: 1) natural flavors, such as artemisia oil, eucalyptus oil, star anise oil, lime oil, white clove extract, clove oil, michelia oil, michelia extract, michelia leaf oil, cedar oil, peppermint oil, atractylodes stearin, clover oil, orange leaf oil, clove basil oil, jasmine extract, wintergreen oil, angelica oil, angelica extract, usnic acid, sandalwood oil, daidai flower oil, daidai leaf oil, hyacinth extract, gum storax, linalyl oil, sassafras oil, sweet pine oil, osmanthus extract, tangerine oil, carrot seed oil, castoreum, ambrette oil, michelia champaca flower oil, agastache rugosus oil, artemisia oil, murraya daniculata concrete, cassie concrete , ginger oil, tangerine oil, peppermint oil, chrysanthemum extract, cumin oil, ambergris, spearmint oil, civet, Winter Sweet oil, Lily of the valley extract, Woody root oil, Aglaia odorata flower oil, rose geranium oil, rose oil, jasmine extract, oyster oil, dark red extract, dark red oil, lemon eucalyptus oil, lemon oil, Qilixiang extract, nan leaf oil, celery rapeseed oil, nutmeg oil, cinnamon oil, litsea oil, mink balm tincture, narcissus extract, pine needle oil, tree moss extract, jasmine extract, musk, musk rat balm, sweet orange oil, sandalwood oil, evening jade oil, evening jade extract, marigold essential oil, foeniculum vulgare oil, sabin oil, citronella oil, geranium oil, carnation oil, perilla oil, perilla oil, cyperus oil, bergamot oil, vetiver oil, elsholtzia oil, elsholtzia extract, rosemary oil, rosemary extract, lavender oil, bay leaf oil, ylang oil, coriander seed oil, fish vanilla oil, rosemary extract, cassia oil, grapefruit peel oil, iris oil, tobacco flower oil, citron leaf oil, gardenia extract, amorpha oil, violet leaf extract, guaiacol, 4-methylguaiacol, 4-ethylguaiacol, natural vanillin, and camphor; 2) synthetic essences such as limonene, longifolene, ethyl caryophyllene, isolongifolene, isolongifolenone, isolongifolanone, beta-bromostyrene, diphenylmethane, diphenylether, m-methyldiphenyl ether, eugenol, isoeugenol, ethylnaphthalene methyl ether, ethylnaphthalene ethyl ether, p-methylanisole, isoeugenol benzyl ether, rose ether, sandalwood ether, 4-Methylphenyl benzyl ether (or jasmine ether), ambrox, methyl cedryl ether, epoxy cedryl, elintal, elintal N, 3-hexenol, decanol, lauryl alcohol, benzyl alcohol, phenylethyl alcohol, p-methoxybenzyl alcohol, cinnamyl alcohol, dimethylbenzyl methanol, geraniol, nerol, linalool, roseol, terpineol, citronellol, racemic menthol, menthol, borneol, cedrol, cedanone, methyl cedrone, 3,4-dioxomethylene benzyl alcohol, α-methyl-3,4-dihydromethylene phenylpropanal, 2-tert-butyl-4-methylcyclohexanol, sclareol, santalol, 3-methyl-5-(2',2',3'-trimethylcyclopenten-1'-yl) pentan-2-ol, benzaldehyde, acetophenone, phenylacetic acid, p-methylacetophenone benzophenone, lauraldehyde, cinnamaldehyde, cinnamic acid, α-pentyl-β-phenylacrolein, A-phenylpropanal, lilial, lyral, cyclamen aldehyde, 3,4-dioxomethylene benzaldehyde, 3,7-dimethyl-6-octenal, 3,7-dimethyl-7-hydroxyoctanal, citral, ethyl-homocitral, vertoliff, myrac, 4-hydroxy-3-methoxybenzaldehyde, alpha-dihydro damascone, beta-damascenone, ionone, 6-methylionone, nerolinone, carvone, piperonyl acetone, raspberry ketone, herbac, cis-jasmone, heptanal ethylene glycol acetal, phenylacetaldehyde dimethyl acetal, octylacetaldehyde dimethyl acetal, alpha-amylcinnamic aldehyde dimethyl acetal, citral dimethyl acetal, citral diethyl acetal, anisaldehyde dimethyl acetal, mushroom aldehyde, phyllocyanin, hyacinth, apple ester, jeep ketal, geranyl formate, citronellyl formate, aphermate, cis-3-Hexenyl acetate, benzyl acetate, phenylethyl acetate, cinnamyl acetate, terpinyl acetate, bornyl acetate, linalyl acetate, geranyl acetate, p-tert-butylcyclohexyl acetate, o-tert-butylcyclohexyl acetate, vetiveryl acetate, succinyl acetate, rosamusk, decahydronaphthyl acetate, cedryl acetate, geranyl propionate, trichloromethyl benzyl acetate, benzyl butyrate, cis-3-hexenyl butanoate, geranyl butyrate, Phenylethyl 2-methylpentanoate, p-cresyl isobutyrate, methyl 2-nonenoate, ethyl benzoate, methyl benzoate, cis-3-Hexenyl benzoate, benzyl benzoate, ethyl phenyl acetate, p-cresyl phenyl acetate, ethyl cinnamate, butyl salicylate, isobutyl salicylate, isoamyl salicylate, Cis-Hexenyl Salicylate, hexyl salicylate, benzyl salicylate, phenethyl salicylate, cyclohexyl salicylate, jasmine, jasmonate, methyl 2-pentylcyclopentanone acetate, aurantiol, γ-nonalactone, γ-undecalactone, coumarin, indole, 3-methylindole, 3,7-dimethyl-2,6-octadiene nitrile, p-methoxybenzonitrile, xylene musk, ketone musk, 2,6-dinitro-3-methoxy-4-tert-butyltoluene, 1,1-dimethyl-4-acetyl-6-tert-butylindane, hexamethyl tricyclic isochroman musk, 1,1,2,3,3,6-hexamethyl-5-acetylindan, musk-T, musk-M, musk-L, musk-F, 11-oxacyclohexadecanolide, and tonal musk; 3) essence, rose essence, jasmine essence, sandalwood essence, and brandy essence.

The pigment may be one or more selected from: zirconium dioxide, lead acetate, silver nitrate, chlorophyll, copper chlorophyll, iron ferrocyanide, guanine, fast red, food red I, vat red I, carmine, edible bayberry red, edible cherry red, food red 17, amaranth red, fast green, food yellow 3, edible lemon yellow, edible sudan yellow, edible indigo, food blue 2, fluorescent peach red, beta-carotene, henna, gardenia red pigment, gardenia green pigment, gardenia blue pigment, gardenia yellow pigment, titanium mica pearlescent pigment, iron oxide red, iron oxide yellow, iron oxide black, chromium oxide green, bismuth oxychloride, basic peach red, honeysuckle R, quinoline yellow, shikonin, lac red pigment, ultramarine, acid red 87, acid green 25, acid fluorescent yellow, acid orange 7, jinguang red 53, lithol rubine BK, lithol rubine 2R, and permanent orange.

The preservative may be one or a combination of components selected from: sorbic acid and salts thereof, propionate, p-hydroxybenzoic acid ester, benzyl alcohol, benzoic acid and salts thereof, 5-chloro-2-methyl-4-isothiazolin-3-one, -2-methyl-4-isothiazolin-3-one, cadimon, imidazolidinyl urea, dehydroacetic acid and salts thereof, sodium sulfite, sodium bisulfite, sodium metabisulfite, 2,2-bromo-2-nitro-1, 3-propanediol (Brobol), erythorbic acid and salts thereof, kojic acid and esters thereof, azelaic acid, usnic acid, and resorcinol.

The antioxidant may be selected from one or a combination of components selected from: SOD, butylated hydroxyanisole, gallic acid ester, ascorbic acid and salts thereof, erythorbic acid and salts thereof, sitosterol, rutin, and tocopherol.

The humectant may be one or a combination of components selected from: 1,2-butanediol, sorbitol, xylitol, glycerol, lactate, polyethylene glycol, DL-pyrrolidone carboxylate, D-glucose, kojic acid and esters thereof, uric acid, orotic acid, pectinic acid, laminin, collagen, trehalose, and cholesteryl 12-hydroxystearate.

The ultraviolet absorber may be one or a combination of components selected from: pyridoxine hydrochloride, kojic acid and esters thereof, rutin, aloin, caffeic acid, hesperetin, 2-ethylhexyl salicylate, 2-ethylhexyl p-dimethylaminobenzoate, 2-ethylhexyl p-methoxycinnamate, phenyl salicylate, anthranilate, 2-hydroxy-4-methoxybenzophenone, 2-hydroxy-4-methoxybenzophenone-5-sulfonic acid, and 2-hydroxy-4-octyloxybenzophenone.

The chelating agent may be one or a combination of components selected from: edta disodium salt, zinc pyrithione, and sodium thiosulfate.

The astringent may be one or a combination of components selected from: zinc phenol sulfonate, aluminum hydroxychloride, aluminum sulfate, potassium aluminum sulfate, zinc sulfate, aluminum chloride, zinc chloride, and caramin.

The penetration enhancer may be one or a combination of components selected from: dimethyl sulfoxide, 1-n-dodecylazepan-2-one, and 2-aminoethanol.

The pH adjusting agent may be one or a combination of components selected from: tartaric acid, lactic acid, boric acid, and succinic acid.

The nutritional additive may be one or a combination of components selected from: chitin, chitosan, silk peptide, silk fibroin, lecithin, epidermal growth factor, trehalose, lactose, pearl powder, collagen, collagen hydrolysate, hyaluronic acid and salts thereof, egg membrane protein, phytosterol, lysozyme chloride, sodium chondroitin sulfate, bee milk, propolis, honey, glycyrrhizic acid, glycyrrhetinic acid, y-linolenic acid, kojic acid and esters thereof, gibberellic acid, glycine, L-aspartic acid and its salts, L-cystine and its salts, DL-alanine, L-serine, DL-serine, L-methionine, DL-methionine, L-lysine hydrochloride, L-threonine, inositol, maltitol, pantothenate, D-panthenol, retinol acetate, allantoin, pyridoxine hydrochloride, cortisone, nicotinamide, vitamin, estrogen, hematoxylin, uric acid, orotic acid, aloin, azelaic acid, abietic acid, usnic acid, laminin, thymosin, alizarin, limonene, baicalein, deoxycholic acid, guaiacus, hesperetin, tannic acid, selenium disulfide, tea saponin, triethyl citrate, butylhydroxytoluene, phytic acid, and dimethyl diallyl ammonium chloride.

### Experimental Examples

The instruments, kits, cells, and reagents used in the experimental examples are available from public commercial channels and are commonly used in the art, and usage of the instruments and kits observes the instructions thereof.

Purpose of the experiment: An oxidative damage model of Hacat cells induced by UVB was established, and effects of cosmetic materials on the expression of NQO1 protein were detected by SOD assay kits and Western Blot. Superoxide dismutase (SOD) catalyzes dismutation of superoxide anions to produce hydrogen peroxide (H₂O₂) and oxygen (O₂), and is an important antioxidant enzyme in organisms. Quinone oxidoreductase 1 (NQO1) is a homodimeric flavo-enzyme, and is capable of preventing oxidative damages to DNA caused by environmental stress agents. In addition, NQO1 plays an important role in protecting endogenous antioxidants by maintaining the reduced forms of ubiquinone and alpha-tocopherolquinone.

### Experimental material

Starting material 1: Curcuma Longa (Turmeric) root extract; trade name: Tego Turmerone; physical property: oily; available from Evonik Specialty Chemicals (Shanghai) Co., Ltd.

Starting material 2: dimethylmethoxy chromanol; trade name: LIPOCHROMAN; physical property: light brown to brown powder; available from Lubrun Management (Shanghai) Co., Ltd.

Main instruments and reagents

**Table 1 Main instruments used in experiment**

| Name | Model/Specification | Manufacturer |
|---|---|---|
| Household refrigerating freezer | BCD-213D11D | Haixin Rongsheng (Guangdong) Refrigerator Co., Ltd. |
| Biological microscope | LWD300-38LT | Shanghai Cewei Photoelectric Technology Co., Ltd |
| Digital display constant temperature water bath kettle | HH-W420 | Guowang Experimental Instrument Factory (Jintan City) |
| Medical low-temperature refrigerator | ULTS1368 | Thermo |
| Blast drying oven | DHG-9075A | Shanghai Yiheng Scientific Instrument Co., Ltd. |
| High speed refrigerated centrifuge | D-37520 | Kendro |
| Autoclave | GI54DS | Zhiwei (Xiamen) Instrument Co., Ltd. |
| CO₂ Incubator | BB 150 | Thermo |
| Pure water instrument | MicroPure | Thermo |
| Double clean bench | SW-CJ-2FD | Suzhou Suhang Technology Equipment Co., Ltd. |
| Microplate reader | MuLtiskan FC | Thermo |
| Ultraviolet cross-linking instrument | Scientz03-II | Ningbo Xinzhi Biotechnology Co., Ltd. |
| Benchtop centrifuge | ST16 | Thermo |
| Pipettor | 10 µL | Eppendorf |
| Pipettor | 200 µL | Eppendorf |
| Pipettor | 1000 µL | Eppendorf |
| Pipettor | 2.5 µL | Eppendorf |
| Eight-channel pipette | 300 µL | Eppendorf |

**Table 2 Main reagents used in experiment**

| Name | Specification | Lot No. | Manufacturer |
|---|---|---|---|
| Dmso (cell culture grade) | 100 mL/vial | 1129E031 | Beijing Solarbio Science & Technology Co., Ltd. |
| Absolute ethanol | 500 mL/vial | 20201109 | Shanghai Lingfeng Chemical Reagent Co., Ltd. |
| Vitamin C | 100 g/vial | 716B031 | Solarbio |
| Mem-EBSS medium | 500 mL/vial | 20201123/20210315/20210803 | Cellmax |
| Serum | 500 mL/vial | 1913445/2053264 | Biological Industries |
| Pancreatin | 500 mL/vial | 20210113/20210518/20210813 | Beyotime |
| Bispecific antibody | 100 mL/vial | 20200828 | Solarbio |
| Total superoxide dismutase assay kit | 100 T | 020821210418/052621210618 | Beyotime |

### Cell culture

Cell species: Hacat human immortalized keratinocytes. Source: Cell Bank of Wuhan University, Committee for Type Culture Collection, Chinese Academy of Sciences. Cell type: adherent cells.

Incubation conditions: 5% CO₂, 37°C constant temperature incubator. Media: 89% MEM+10% FBS+1% bispecific antibody.

Preparation method of a test substance and a control substance: The test substance and the control substance were prepared into a mother liquid using DMSO or ddH₂O, and then the mother liquid was diluted using serum-free medium or PBS to appropriate concentration, and finally the following experimental group was obtained:

**Table 3 Experiment group**

| Group | Administration composition (mass fraction ratio) | Administration concentration |
|---|---|---|
| Blank control group | \ | \ |
| Model group | \ | \ |
| Single administration group 1 | Lipochroman | 20 µg/mL |
| Single administration group 2 | Curcuma Longa (Turmeric) root extract | 20 µg/mL |
| Experimental group 1 | Curcuma Longa (Turmeric) root extract: lipochroman=1:1 | 20 µg/mL |
| Experimental group 2 | Curcuma Longa (Turmeric) root extract: lipochroman=2:1 | 20 µg/mL |
| Experimental group 3 | Curcuma Longa (Turmeric) root extract: lipochroman=1:5 | 20 µg/mL |

### Test method

### (I) Antioxidant enzyme assay

1. Test procedures: A single cell suspension was prepared using cells in exponential growth phase; and the cells were inoculated on a 6-well plate at a density of 1.5×10⁵ cells/mL, and cultured at 37°C for 24 h using a medium containing 10% fetal bovine serum to make the cells adhere to each other, with a final culture volume of 2 mL. The supernatant was then suctioned and discarded, and incubated in a serum-free medium or a freshly prepared serum-free medium containing different concentrations of the test substance, with a final culture volume of 2 mL. After incubation for 12 h/16 h/24 h/32 h/48 h/72 h, the cells were subjected to UVB irradiation (50 J/cm², 5 min) using an ultraviolet lamp, and after incubation for 16 h at 37°C, the SOD activity was detected using the superoxide dismutase assay kit from Beyotime.
   The superoxide dismutase assay kit (Total Superoxide Dismutase Assay Kit with WST-8 method) from Beyotime is an assay kit for colorimetric detection of SOD, i.e., superoxide dismutase activity in cells, tissues or other samples based on WST-8 chromogenic reaction. For the specific operation steps, reference may be made to the instructions for use of the assay kit.
2. Data entry and statistical analysis: Statistical processing was performed using Excel entry and the Prism (Version7) software package, expressed as mean±standard error. One-way ANOVA was performed between groups and p<0.05 was considered statistically significant.
3 Experiment results

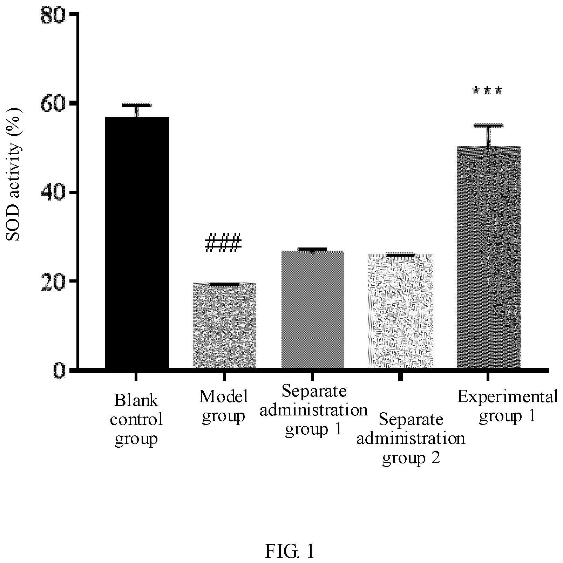

FIG 1 is a graph of comparison of effects of a blank control group, a model group, a separate administration group 1, a separate administration group 2, and an experimental group 1 on UVB-induced SOD activity of Hacat cells.

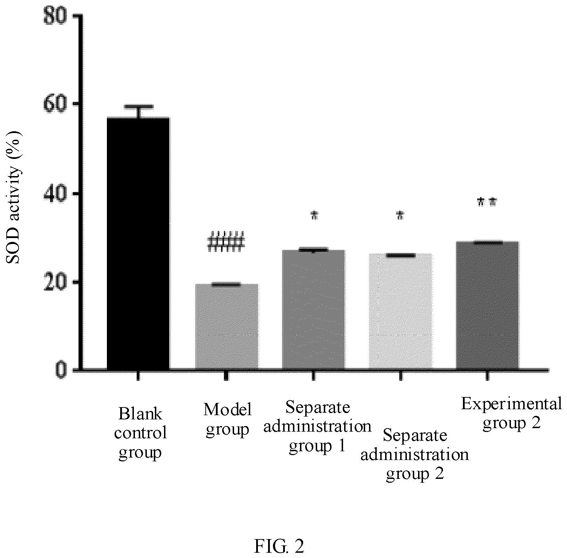

FIG 2 is a graph of comparison of effects of a blank control group, a model group, a separate administration group 1, a separate administration group 2, and an experimental group 2 on UVB-induced SOD activity of Hacat cells.

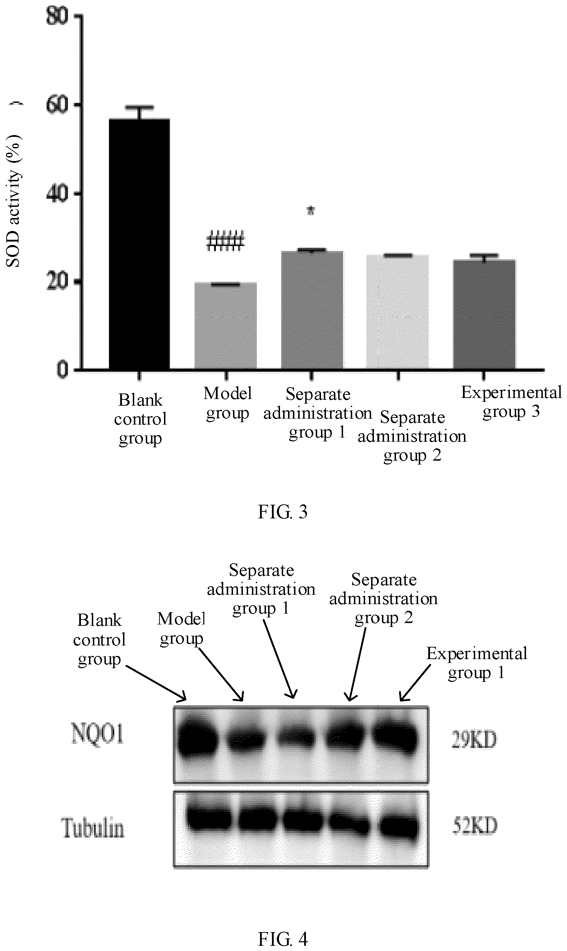

FIG 3 is a graph of comparison of effects of a blank control group, a model group, a separate administration group 1, a separate administration group 2, and an experimental group 3 on UVB-induced SOD activity of Hacat cells.

In FIG 1, n =3, Mean±SEM. "###" means p<0.001 compared to the control group, "*" means p<0.05 compared to the model group, "**" means p<0.01 compared to the model group, and "***" means p<0.001 compared to the model group.

According to FIG 1, the experimental group 1 was administered (in parts by mass, Curcuma Longa (Turmeric) root extract:lipochroman=1:1); and compared to the separate administration group 1 (lipochroman alone) and the separate administration group 2 (Curcuma Longa (Turmeric) root extract alone), the experimental group 1 significantly increased the SOD activity of Hacat cells, indicating that the experimental group 1 achieved a synergistic effect in promoting the expression of antioxidant enzymes.

According to FIG 2, the experimental group 2 was administered (in parts by mass, Curcuma Longa (Turmeric) root extract:lipochroman=2:1); and compared to the separate administration group 1 (lipochroman alone) and the separate administration group 2 (Curcuma Longa (Turmeric) root extract alone), the experimental group 2 also significantly improved the SOD activity of Hacat cells.

According to FIG 3, the experimental group 3 was administered (in parts by mass, Curcuma Longa (Turmeric) root extract:lipochroman=L5); and compared to the separate administration group 1 (lipochroman alone) and the separate administration group 2 (Curcuma Longa (Turmeric) root extract alone), the experimental group 3 achieved no significant effect in improving the SOD activity of Hacat cells.

### (II) NQO1 protein expression detection

1. Test procedures: A single cell suspension was prepared using cells in exponential growth phase; and the cells were inoculated on a 6-well plate at a density of 1.5×10⁵ cells/mL, and cultured at 37°C for 24 h using a medium containing 10% fetal bovine serum to make the cells adhere to each other, with a final culture volume of 2 mL. The supernatant was then suctioned and discarded, and incubated in a serum-free medium or a freshly prepared serum-free medium containing different concentrations of the test substance, with a final culture volume of 2 mL. After incubation for 12 h/16 h/24 h/32 h/48 h/72 h, the cells were subjected to UVB irradiation (50 J/cm², 5 min) using an ultraviolet lamp, and after incubation for 16 h at 37°C, the NQO1 protein content was detected using Western Blot.

Total protein extraction method: Lysate was added to the 6-well plate, and lysed on ice for 30 min; and the cells were scraped off with a cell scraper and transferred to a centrifuge tube. The cells were centrifuged 5 min at 12000 rpm at 4°C, the supernatant was placed in a new centrifuge tube, a 5x loading buffer was added according to the volume of the supernatant, the centrifuge tube was boiled in boiling water for 10 min for protein denaturation, the protein concentration was measured after cooling, and the tube was placed at -80°C for storage.

Protein concentration measurement by BCA method: The test was carried out according to the instructions of the assay kit. (BCA assay kit brand: Biosharp)

Western blot (NQO1 antibody, CST): Protein loading was subjected to SDS-PAGE gel electrophoresis; after electrophoresis, the protein bands were transferred to an NC membrane; after blocking with 5% skim milk, the primary antibody was incubated overnight at 4°C; after washing the membrane, the secondary antibody was incubated for 1 h; and finally, the band was developed using an ECL substrate luminescence solution.

2. Data entry and statistical analysis: Statistical processing was performed using Excel entry and the Prism (Version7) software package, expressed as mean±standard error. One-way ANOVA was performed between groups and p<0.05 was considered statistically significant.

3 Experiment results

FIG 4 is a WB graph of NQO1 protein expression of UVB-induced Hacat cells upon administration in a blank control group, a model group, a separate administration group 1, a separate administration group 2, and an experimental group 1.

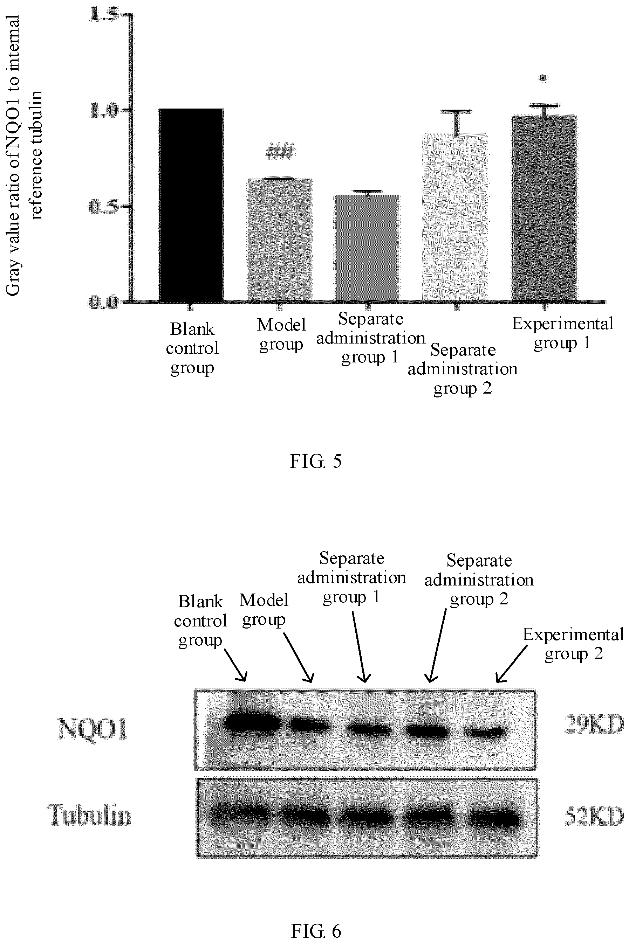

FIG 5 is a graph of comparison of effects of a blank control group, a model group, a separate administration group 1, a separate administration group 2, and an experimental group 1 on NQO1 protein expression of UVB-induced Hacat cells.

FIG 6 is a WB graph of NQO1 protein expression of UVB-induced Hacat cells upon administration in a blank control group, a model group, a separate administration group 1, a separate administration group 2, and an experimental group 2.

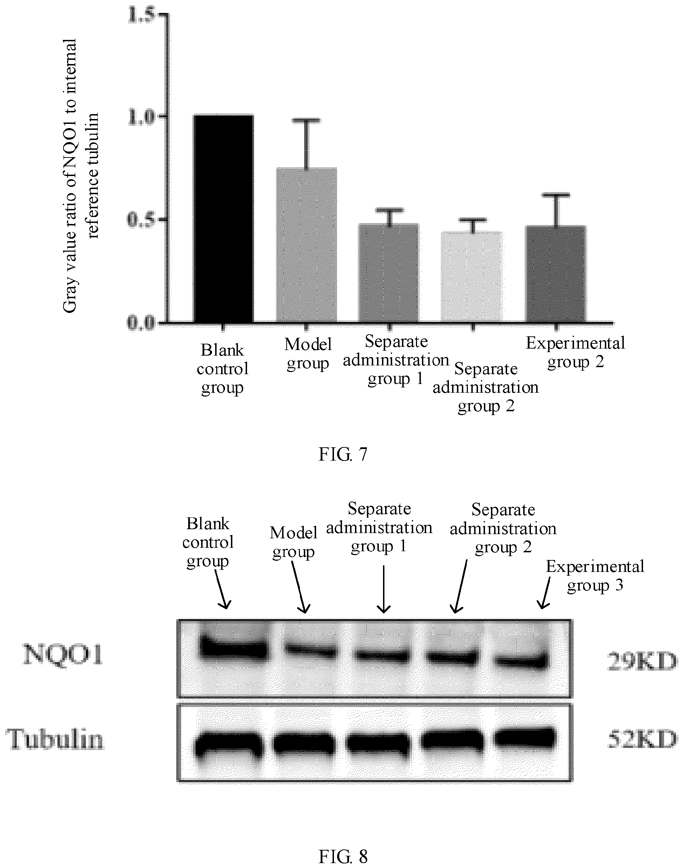

FIG 7 is a graph of comparison of effects of a blank control group, a model group, a separate administration group 1, a separate administration group 2, and an experimental group 2 on NQO1 protein expression of UVB-induced Hacat cells.

FIG 8 is a WB graph of NQO1 protein expression of UVB-induced Hacat cells upon administration in a blank control group, a model group, a separate administration group 1, a separate administration group 2, and an experimental group 3.

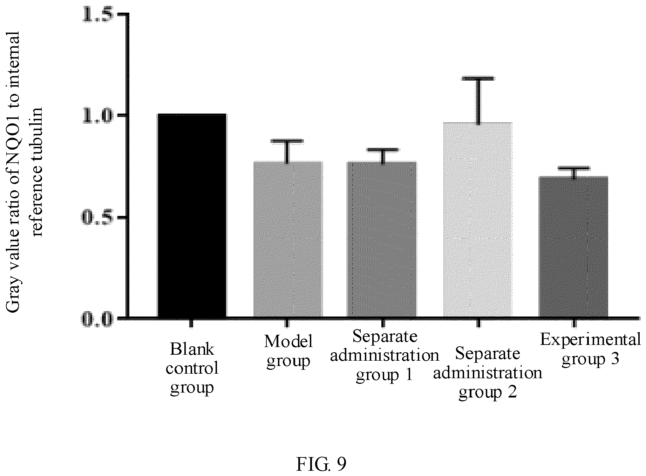

FIG 9 is a graph of comparison of effects of a blank control group, a model group, a separate administration group 1, a separate administration group 2, and an experimental group 3 on NQO1 protein expression of UVB-induced Hacat cells.

n =3, Mean±SEM. "#" means p<0.05 compared to the control group, "##" means p<0.01 compared to the control group, "*" means p<0.05 compared to the model group.

According to FIG 4 and FIG 5, the experimental group 1 was administered (in parts by mass, Curcuma Longa (Turmeric) root extract:lipochroman=1:1); and compared to the separate administration group 1 (lipochroman alone) and the separate administration group 2 (Curcuma Longa (Turmeric) root extract alone), the experimental group 1 significantly increased the NQO1 protein expression of Hacat cells.

According to FIG 6 and FIG 7, the experimental group 2 was administered (in parts by mass, Curcuma Longa (Turmeric) root extract:lipochroman=1:5); and compared to the separate administration group 1 (lipochroman alone) and the separate administration group 2 (Curcuma Longa (Turmeric) root extract alone), the experimental group 2 achieved no significant effect in improving the NQO1 protein expression of Hacat cells.

According to FIG 8 and FIG 9, the experimental group 3 was administered (in parts by mass, Et-VC:VC-IP:VE:NAC=2:3:0.5:0.01); and compared to the separate administration group 1 (lipochroman alone) and the separate administration group 2 (Curcuma Longa (Turmeric) root extract alone), the experimental group 3 achieved no significant effect in improving the NQO1 protein expression of Hacat cells.

Persons of ordinary skill in the art shall understand that the above embodiments are merely specific and exemplary embodiments for practicing the present disclosure, and in practice, various modifications may be made to these embodiments in terms of formality and detail, without departing from the spirit and scope of the present disclosure.

## Claims

1. A composition capable of enhancing an expression efficacy of an antioxidant enzyme, comprising: a Curcuma Longa (Turmeric) root extract and dimethylmethoxy chromanol, wherein a mass fraction ratio of the Curcuma Longa (Turmeric) root extract to the dimethylmethoxy chromanol is (0.5-3): 1.

2. The composition according to claim 1, wherein the mass fraction ratio of the Curcuma Longa (Turmeric) root extract to the dimethylmethoxy chromanol is (1-2): 1.

3. The composition according to claim 1, wherein the mass fraction ratio of the Curcuma Longa (Turmeric) root extract to the dimethylmethoxy chromanol is 1:1.

4. Use of the composition capable of enhancing the expression efficacy of the antioxidant enzyme according to any one of claims 1 to 3 in a cosmetics product and/or a skin care product.

5. The use according to claim 4, wherein the cosmetics product and/or the skin care product is applied to an outer surface of skin.

6. The use according to claim 4, wherein the cosmetics product and/or the skin care product is selected from one or more of eye cream, face cream, essence, lotion, and mask essence.

7. The use according to claim 4, wherein the composition is dispersed as an additive in a base material selected from one or more of an oily material, a powdery material, and a colloidal material.

8. The use according to claim 4, wherein the cosmetics product and/or the skin care product further comprises: one or more of a surfactant, a perfume and fragrance, a pigment, a humectant, a preservative, an antioxidant, a UV absorber, a chelating agent, an astringent, a penetration enhancer, and a nutritional additive.
